# EUROPEAN PATENT SPECIFICATION

(11) **EP 3 154 600 B1**
(45) Date of publication and mention of the grant of the patent: **29.05.2019**
(21) Application number: 15806752.0
(22) Date of filing: 12.06.2015
(51) Int. Cl.: A61L 27/36, A61L 31/04, A61F 2/02

(54) **ARTIFICIAL BIOMEMBRANE USING COCOON AND METHOD FOR MANUFACTURING SAME**
KÜNSTLICHE BIOMEMBRAN MIT VERWENDUNG EINES KOKONS UND VERFAHREN ZUR HERSTELLUNG DAVON
BIOMEMBRANE ARTIFICIELLE UTILISANT UN COCON ET SON PROCÉDÉ DE FABRICATION

(30) Priority: 13.06.2014 KR 20140071972
(43) Date of publication of application: 19.04.2017
(73) Proprietor: Republic of Korea Management: Rural Development Administration, Jeonju-si, Jeollabuk-do 560-500 (KR)
(72) Inventor: JO, You Young, Wanju-gun Jeollabuk-do 565-851 (KR); KWEON, Hae Yong, Suwon-si Gyeonggi-do 443-747 (KR); LEE, Kwang Gill, Suwon-si Gyeonggi-do 441-390 (KR); YEO, Joo Hong, Suwon-si Gyeonggi-do 443-709 (KR); LEE, Heui Sam, Suwon-si Gyeonggi-do 443-709 (KR); KIM, Hyun Bok, Wanju-gun Jeollabuk-do 565-851 (KR)
(74) Representative: Arends, William Gerrit
(86) International application number: PCT/KR2015/005925
(87) International publication number: WO 2015/190860

(56) References cited:
- JP-A- H06 166 850
- JP-A- H06 166 850
- KR-A- 20030 097 691
- KR-A- 20060 038 096
- KR-A- 20100 121 169
- US-A1- 2004 097 709
- JO, Y. Y. ET AL. J. SERIC. ENTOMOL. SCI. vol. 51, no. 1, 2013, pages 68 - 72, XP008185434

## Description

### Technical Field

The present invention relates to an artificial biomembrane using cocoon, and a manufacturing method thereof. More particularly, the present invention relates to a cocoon-based, artificial biomembrane that is biocompatible and has excellent cell growth potential, and a method for manufacturing the same.

Also, the present invention relates to a biomembrane that has excellent tensile strength and elongation, and a manufacturing method therefor.

### Background Art

Of various materials for biomedical applications, animal-derived collagen is of high biocompatibility and histocompatibility. In addition, collagen has hemostatic activity, and is biodegradable and completely absorbed in vivo.

Such collagen can be isolated and purified from connective tissues of various animal organs, including skin, bone, cartilage, tendon, etc. by acidolysis, alkalinolysis, neutral hydrolysis, or enzymolysis.

Conventionally extracted collagens for biomedical materials have a molecular level of monomers or oligomers and are stored in the form of powder or liquid.

Since their collagen molecules are decomposed to the degree of monomers or oligomers, conventionally extracted collagens rapidly becomes sol upon exposure to water, body fluid, or blood.

To be useful as a biomedical material in the body, collagens need hardness to some degree. For this, collagens are applied to synthetic polymer materials such as nylon, silicon, etc. Additionally or alternatively, a graft made of extracted collagen is cured chemically with a crosslinking agent or physically with radiation, such as electron radiation or UV radiation, or heat in order to maintain its form for a predetermined period of time after application to the body.

However, when collagen is combined with synthetic polymer materials, the synthetic polymer materials remain as foreign matter in the body after the degradation of the collagen, and thus are apt to cause side effects such as bulbils generation, inflammation, etc. Thus, synthetic polymer materials cannot be applied to any cell or organ.

Though collagen materials are crosslinked, their physical properties, especially tensile strength, do not significantly improve. Hence, collagen, although subjected to a processing treatment, is impossible to use as biomedical material requiring suturing.

In addition, a crosslinking agent, such as glutaraldehyde or epoxy, when used alone, may not only exert toxicity to the body, but also degrade the collagen's intrinsic biochemical properties, especially promotive effects on cell growth.

Physical crosslinking does not guarantee sufficient physical properties to the collagen graft. With physical crosslinking, the collagen is also difficult to endow with a proper absorption rate in vivo.

After a surgical operation is performed on various organs including the brain to treat various diseases or injuries, the surgical site, e.g., dura mater, pericardium, pleura, peritoneum, or serosa, must or may be sealed by suturing. Because shrinkage occurs at the sutured site or the membrane is at least partially dissected, the surgical site cannot be completely sealed and the membrane is, for the most part, defected.

When the defect is neglected, either the organ, such as the brain, the heart, the lung, the intestine, etc., may stick out of the defect leading to more serious complications, or the organ or its surrounding area is exposed to water or air, which disturbs the healing of surgical site.

In order to overcome these problems, conventionally, lyophilized human cadaver dura mater, or an expanded polytetrafluoroethylene (EPTFE) film, a polypropylene mesh, a Teflon sheet or a Dacron sheet is used as a complement for the defect.

However, these conventional artificial biomembranes, when used, for example, in human dura mater, may cause adhesion with the cerebral parenchyma, which may result in the onset of post-operative epilepsy. Particularly, EPTFE films do not degrade, but remain as foreign materials that are likely to mediate infection. Further, when tissues are in contact with the EPTFE films, cells undergo fatty degradation. As such, the conventional artificial biomembranes are prone to causing post-operative complications.

Now, research has been directed toward the development of materials for biomembranes that can be sutured while retaining biochemical properties and which can maintain intended shapes for a predetermined period of time after in vivo application.

With regard to related art, reference may be made to Korean Patent Unexamined Publication Application No. 10-2002-0036225 (issued on May 16, 2002, titled "Biomembrane Dressing for Healing Dermal Wound and Infection") published as KR20020036225 and Korean Patent No. 10-1280722 (issued on June 25, 2013, titled "Thin film multilocular structure made of collagen, member for tissue regeneration containing the same, and method for producing the same").

JP H06 166850 discloses a method for forming a harmless practical transparent homogeneous thin film from a cocoon solution. The method comprises dissolving silkworm cocoon in a haloacetic acid. A multilayered fibrous membrane forming a cocoon is peeled off, solubilized with the acid and then solidified to form a thin film.

### Disclosure of Invention

### Technical Problem

It is an object of the present invention to provide a biocompatible silkworm cocoon-based biomembrane that can be relatively simply manufactured in a more cost efficient manner than conventional artificial biomembranes and which has excellent cell growth potential, and a method for manufacturing the same.

It is another object of the present invention to provide a silkworm cocoon-based artificial biomembrane having excellent tensile strength and elongation, and a method for manufacturing the same.

Embodiments of the present invention will be described in detail with reference to the accompanying drawings. These embodiments will be described in detail in order to allow those skilled in the art to practice the present invention. It should be appreciated that various embodiments of the present invention are different, but are not necessarily exclusive.

### Solution to Problem

In order to accomplish the above objects, an aspect of the present invention provides a silkworm cocoon-based artificial biomembrane as described in claims 1-9. The silkworm cocoon-based artificial biomembrane is prepared by dividing a cocoon having a first shell thickness into two or more fragments in a predetermined form.

In one exemplary embodiment of the present invention, each of the fragments is delaminated into a lamellar fragment with a second thickness, the second thickness being smaller than the first thickness.

In another exemplary embodiment of the present invention, the lamellar fragment with a second thickness is an inner, mid or outer stratum of the cocoon.

In another exemplary embodiment of the present invention, the lamellar fragment is sterilized or packed.

In accordance with another aspect thereof, the present invention provides a method according to claim 10 for manufacturing a silkworn cocoon-based artificial biomembrane comprising a fragment of a silkworm cocoon, comprising a first step of dividing a cocoon into two or more fragments in a predetermined form, the cocoon having a shell with a first thickness.

In one exemplary embodiment of the present invention, the method may further comprise a second step of delaminating each of the fragments into a lamellar fragment with a second thickness, the second thickness being smaller than the first thickness.

In another exemplary embodiment of the present invention, the method may further comprise a third step of packing the fragments of the second thickness prepared in the second step.

In another exemplary embodiment of the present invention, the method may further comprise conducting sterilization before or after each step.

In another exemplary embodiment of the present invention, the lamellar fragment with a second thickness is an inner, mid or outer stratum of the cocoon.

### Advantageous Effects of Invention

The artificial biomembrane of the present invention is biocompatible not only because it has high tensile strength and elongation, which are necessary for biomembranes, but also because it exhibits excellent cell growth potential. In addition, the artificial biomembrane can be prepared easily and thus in a cost-efficient manner, compared to conventional artificial biomembranes.

### Brief Description of Drawings

FIG. 1 is a schematic view illustrating a manufacturing procedure of a cocoon-based artificial biomembrane according to one embodiment of the present invention;
FIG. 2 is a schematic view illustrating the manufacturing procedure of a cocoon-based artificial biomembrane according to a modified embodiment of the present invention;
FIG. 3 shows morphologies of cocoon fragments used in the artificial biomembranes of the present invention;
FIG. 4 is a graph showing a mechanical property (tensile strength) of the cocoon-based artificial biomembrane of the present invention; and
FIG. 5 is a graph showing the cell growth potentials of the cocoon-based artificial biomembranes of the present invention.

### Best Mode for Carrying out the Invention

Unless otherwise specified, the terms and techniques used in the specification should be defined to have the same meanings as are generally accepted in the art to which the present invention pertains.

Unless otherwise defined, all terms used herein have the same meaning as commonly understood by one of ordinary skill in the art to which example embodiments belong. It will be further understood that terms, e.g., those defined in commonly used dictionaries, should be interpreted as having a meaning that is consistent with their meaning in the context of the relevant art and will not be interpreted in an idealized or overly formal sense unless expressly so defined herein.

The term "biomembrane" or "biological membrane", as used herein, refers to an enclosing or separating membrane within living things, such as cell membranes, and organelle membranes. That is, serving as a selectively permeable barrier to an external environment, a biomembrane functions to prevent the external release of nucleic acids, proteins and other biomolecules and to construct an independent space where various biological activities happen. Further, a cell membrane protects the cell from external factors, and plays as a passage for material transport between the cytoplasm and the external environment.

For use as an alternative to a biomembrane, an artificial biomembrane has to maintain the intended shape thereof for a predetermined period of time after application to the body. Importantly, the artificial biomembrane must avoid adhesion with tissues around the surgical site after surgical operation, mediation of infection, and tissue degeneration, and must promote regeneration.

Artificial biomembranes find applications in various fields including artificial neural tubes, artificial cord cervical spines, artificial esophagus, artificial bronchi, artificial vessels, artificial valves, artificial dura mater, and artificial ear drums.

For obtaining these properties, artificial biomembranes used thus far need pretreatment processes, such as physical crosslinking, etc. However, such pretreated artificial biomembranes may be toxic to the body and remain as foreign matter upon long-term use in vivo. Moreover, another disadvantage is that the pretreatment processes and chemicals used therefor increases the production cost of the artificial biomembranes.

Leading to the present invention, intensive and thorough research into an artificial biomembrane, conducted by the present inventor, resulted in the finding that a fragment prepared from a cocoon is biocompatible and useful as a biomembrane not only because it has high tensile strength and elongation but also it exhibits an excellent cell growth potential, and that the fragment can be produced at significantly lower cost, compared to conventional artificial biomembranes.

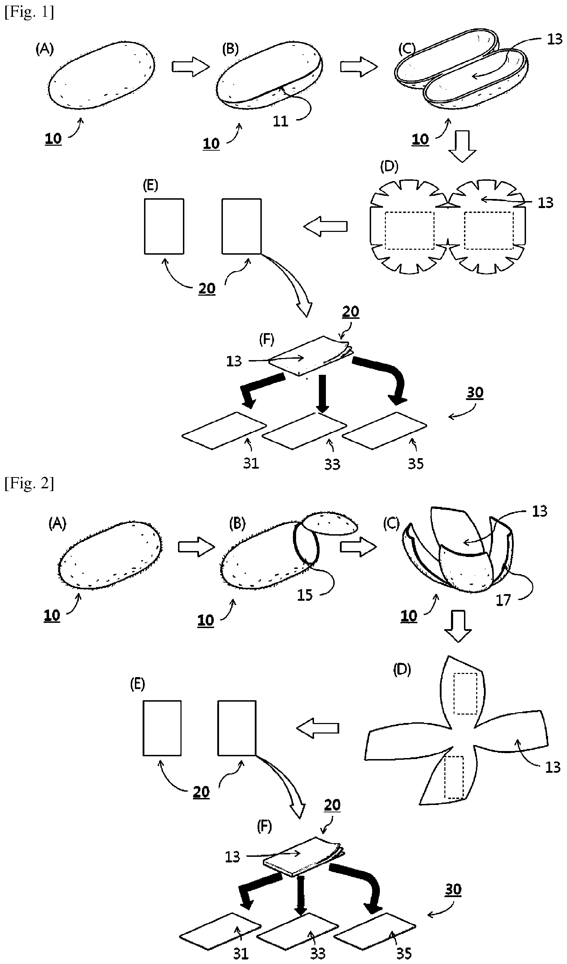

With reference to FIG. 1, a method for manufacturing a cocoon-based artificial biomembrane in accordance with the present invention is explained, below.

### 1. Step 1: Preparation of cocoon fragment with first thickness.

As shown in FIG. 1A, a cocoon 10, the shell of which has a first thickness, is prepared.

A cocoon is a casting spun of silk by silkworms and is used as a material of silk fibers. In the present invention, cocoons, which may be idle resources, are up-cycled into a new high added-value product, thus bringing economic benefits to silkworm farmers.

Naturally constructed by silkworms, which eat clean mulberry leaves, cocoons are free of toxicity and are suitable for use as an environment-friendly material.

Hence, the present invention takes a cocoon 10 as a material for an artificial biomembrane. The cocoon 10 is processed, as shown in FIGS. 1B to 1E, into two or more planar fragments, each having a first thickness.

In greater detail, the oval cocoon 10 is dissected along a cutting line 11 into halves, as shown in FIG. 1B. The dissected halves have semi-oval shapes, and are opened to expose the inside surface 13 of the cocoon, as shown in FIG. 1C.

Next, the cocoon halves with curved inside surfaces 13 are planarized to some degree by cutting many sites along the edge as shown in FIG. 1D, and planar regions are cut out to obtain cocoon fragments with a first thickness, as shown in FIG. 1E.

The artificial biomembrane prepared in the present invention needs not have a planar surface. Because a cocoon originally has an elliptical ball shape, the curved shape of the dissected cocoon may be utilized to give curved artificial biomembranes if necessary. For use as a small artificial biomembrane, a cocoon fragment with a small area may be relatively planar. In contrast, when taken a relatively large area of the dissected cocoon halves, the artificial biomembranes may have curved surfaces.

With reference to FIG. 2, a modified method for preparing a cocoon fragment with a first thickness is described. In detail, an oval-shaped cocoon 10 is cut along cutting lines 15 and 17 to expose the inside 13 of the cocoon 10, as shown in FIGS. 2B and 2C. Then, the dissected cocoon with a curved surface is spread as shown in FIG. 2D, and then cocoon fragments 20 with a first thickness are obtained as shown in FIG. 2E.

The preparation methods of cocoon fragments described in FIGS. 1 and 2 are only illustrative, but are not intended to limit the manufacturing method of artificial biomembranes according to the present invention. A cocoon fragment with a first thickness may be prepared by cutting a cocoon in the manners shown in FIGS. 1 and 2, but other cutting methods may be used.

### 2. Step 2: Preparation of cocoon fragment with second thickness (artificial biomembrane)

Because the cocoon fragments 20 with a first thickness, prepared in step 1, has a multilayer structure identical to that of the cocoon shell, the multilayer structure may be split into thinner layers for use as an artificial biomembrane.

Although the cocoon fragment 20 with a first thickness, prepared in step 1, is itself possible to be used as an artificial biomembrane, it is subjected to thickness splitting to give cocoon fragments 30 with a second thickness. In this regard, the second thickness is smaller than the first thickness.

As they are, the cocoon fragments 30 can be applied for any purpose of artificial biomembranes. If necessary, they may be sterilized or chemically treated.

In detail, a cocoon shell varies in thickness (first thickness) from 0.3 to 1.0 mm depending on silkworm species.

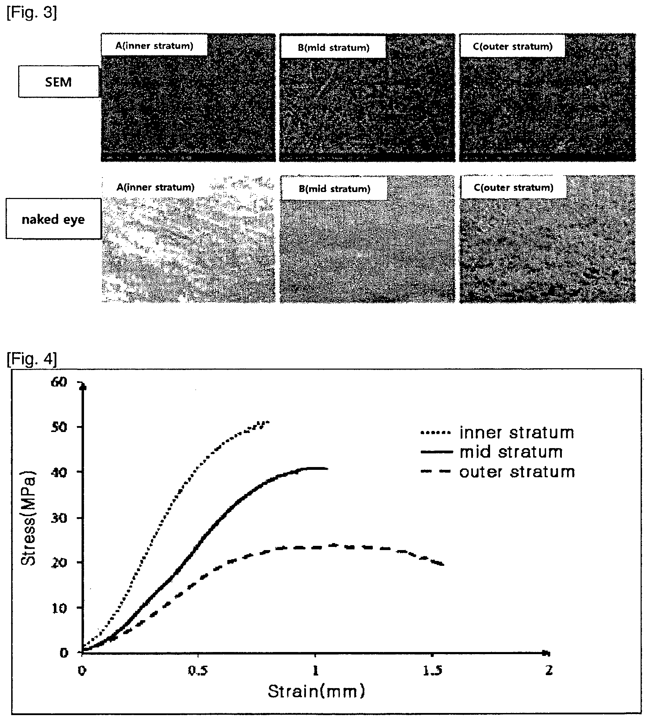

In principal, any kind of cocoons may be used in the present invention. For the purpose of the present invention, a cocoon with a shell thickness of 0.5∼0.8 mm is employed. In the present invention, the cocoon shell is divided into inner, mid and outer strata. First, the outer stratum is defined as a layer that is deep from the outer surface to a point corresponding to 25 % of the total shell thickness. The inner stratum is defined as a layer that is deep from the inner surface to a point corresponding to 15 % of the total shell thickness. The other part corresponding to 60 % of the total shell thickness, that is, the remaining middle cocoon shell except the outer and the inner layer is the mid stratum. The numerical values of the interlayer borders are determined according to characteristics of individual strata (inner, mid, and outer), as shown in FIG. 3. That is, the outer stratum that is deep from the outer surface to a point corresponding to 25 % of the total shell thickness can be used as a cocoon fragment characterized by high elongation. The mid stratum that has a thickness corresponding to 60 % of the total shell thickness exhibits high cell growth potential thanks to its high porosity. The inner stratum that accounts for the remaining 15 % of the total shell thickness has a smooth surface and high tensile strength.

A cocoon fragment can be easily delaminated into up to 16 lamellas although the number of delaminations is dependent on the shell thickness. The thicknesses of the lamellas can be determined according to strength and elongation necessary for the kind and use of the artificial membrane. From a cocoon with a shell thickness of 0.5∼0.8 mm, an artificial biomembrane 0.01mm ∼ 0.7mm thick can be prepared by delamination. According to the use of the artificial biomembrane, selection may be made of the cocoon fragments 30 with various thicknesses.

As can be seen FIG. 4, the outer stratum 35 is suitable as an elastic biomembrane because it is higher in elongation rate than the inner stratum 31 and the mid stratum 33. The highest elongation rate is measured from the outer stratum, with the lowest elongation rate from the inner stratum.

The inner stratum 31 has a smooth surface so that it is unlikely to adhere. In addition, the inner stratum has high tensile strength so that it is suitable for use as a biomembrane where strength is needed. The highest tensile strength is measured in the inner stratum, with the lowest tensile strength in the outer stratum.

Further, the mid stratum 33 exhibits has excellent cell growth potential because of its high porosity. Hence, it is suitably applied as a biomembrane to a defect lesion that needs fast healing.

### Mode for the Invention

A better understanding of the present invention may be obtained through the following examples that are set forth to illustrate, but are not to be construed as limiting the present invention.

### <EXAMPLE 1> Preparation of Artificial Biomembrane 1

A cocoon 10 was prepared, and cut at a proper site to exposure the inside thereof.

Next, the cut cocoon was further processed to make the curved inside planar.

The planarized cocoon was cut into rectangular fragments 20.

From the cocoon fragments 20, a layer containing the innermost surface 13, that is, an inner stratum was delaiminated at a thickness of 0.07 mm.

The delaminated inner stratum was sterilized and used as an artificial biomembrane 1.

### <EXAMPLE 2> Preparation of Artificial Biomembrane 2

From the remaining cocoon after the inner stratum was delaminated in Example 1, a layer containing the outermost surface, that is, an outer stratum opposite to the inner stratum was delaminated at a thickness of 0.07 mm.

The delaminated outer stratum was sterilized and used as an artificial biomembrane 2.

### <EXAMPLE 3> Preparation of Artificial Biomembrane 3

The remainder after the inner stratum and outer stratum were delaminated into mid strata, each 0.07 mm thick, followed by sterilization to give an artificial biomembrane 3.

### <TEST EXAMPLE 1> Morphology of Artificial Biomembranes from Cocoon

### 1. Test Method

Morphologies of the artificial biomembranes prepared in Examples 1 to 3 were observed by scanning electron microscopy (SEM) and with the naked eye. The results are shown in FIG. 3.

### 2. Test Results

As can be seen in FIG. 3, the inner stratum (A, Example 1), the mid stratum (B, Example 3), and the outer stratum (C, Example 2) were different from one another in terms of morphological properties, such as fiber thickness, pore form, etc. Under the naked eye, the mid stratum was observed to have a smoother surface. Thus, the morphological results indicate that the inner stratum, the mid stratum, and the outer stratum can be used where their unique characteristics are needed.

For example, as shown in FIG. 3, the inner stratum has a smooth surface and is water resistant so that it is suitably used as an artificial biomembrane where a non-sticky property is needed. Having high cell growth potential thanks to the high porosity thereof, the mid stratum can be suitably used as a biomembrane in a defect region that needs fast healing.

### <TEST EXAMPLE 2> Physical Properties of Cocoon-Based Artificial Biomembrane According to Cocoon Stratum

### 1. Test Method

Physical properties of the cocoon-based artificial biomembranes prepared in Examples 1 to 3 by cocoon stratum were measured. In this regard, a tensile test was conducted using a universal testing machine (DAEYEONG, Korea).

Test specimens with sizes of 2.5 x 0.07 (width x length) mm were used. The specimens were extended at a rate of 10 mm/min, with an initial gauge length set to be 10 mm.

Results are given in FIG. 4 (strain (mm) versus stress (MPa)) and Table 1, below.

### 2. Test Results

### Table 1

**[Table 1]**

| | Tensile Strength (MPa) | Elongation (%) |
|---|---|---|
| Inner Stratum (Ex. 1) | 60.20±5.3 | 12.45±1.5 |
| Mid Stratum (Ex. 3) | 46.19±2.2 | 15.05±1.7 |
| Outer Stratum (Ex. 2) | 29.36±3.1 | 18.93±1.3 |

As is understood from the data of FIG. 4 and Table 1, the cocoon-based artificial biomembranes were different from one another in tensile strength and elongation by stratum. The highest tensile strength was detected in the inner stratum while the highest elongation was measured from the outer stratum.

In other words, the inner stratum is suitable for use as an artificial biomembrane in a site where strength is important whereas the outer stratum is preferably applied to a site that needs elasticity.

### <TEST EXAMPLE 3> Physical Properties of Mid Cocoon Stratum-Based Artificial Biomembrane According to Thickness

### 1. Test Method

Physical properties of the mid stratum-based artificial biomembranes were measured according to thickness. In this regard, a tensile test was conducted using a universal testing machine (DAEYEONG, Korea). Test specimens with sizes of 20 x 2.5 (width x length) mm were used in a dry state or a wet state. In the latter case, the specimens were immersed in physiological saline for 1 hrs. For a control, a commercially available collagen membrane was employed. The specimens were extended at a rate of 10 mm/min, with an initial gauge length set to be 10 mm. Results are given in Table 2, below.

### 2. Test Results

### Table 2

**[Table 2]**

| Kind | Thick.(m m) | Max. Load (N) | | Tensile Strength (MPa) | | Elongation (%) | |
|---|---|---|---|---|---|---|---|
| | | Dry | Wet | Dry | Wet | Dry | Wet |
| Mid Stratum | 0.01 | 6 | 3 | 2 | 13 | 2 | 23 |
| | 0.02 | 9 | 8 | 2 | 20 | 1.4 | 29.4 |
| | 0.04 | 17 | 11 | 4 | 14 | 1.8 | 23 |
| | 0.06 | 16 | 16 | 4 | 13 | 1.6 | 31.8 |
| | 0.08 | 33 | 15 | 8 | 9 | 2.4 | 25.2 |
| | 0.1 | 37 | 20 | 9 | 10 | 2 | 30.2 |
| | 0.2 | 88 | 62 | 22 | 16 | 2.8 | 26.6 |
| | 0.4 | 113 | 80 | 28 | 10 | 2.8 | 31.6 |
| Collage n | 0.2 | 3.3 | 0.25 | 0.8 | 0.05 | 7.8 | 18.8 |

As is understood from the data of Table 2, the cocoon-based artificial biomembrane differs in tensile strength and elongation by thickness. Both the tensile strength and the elongation increased with the increase of thickness, and were better in a wet state than in a dry state. In contrast, the tensile strength of the commercially available collagen membrane decreased 16 times when it was in a wet state compared to when it was in a dry state. Taken together, the data indicates that physical properties of the cocoon-based biomembranes can be maintained for a longer period of time than those of the wet collagen membrane.

### <TEST EXAMPLE 4> Cell Growth Potential of Artificial Biomembrane

### 1. Test Method

In order to test the artificial biomembranes for cell growth potential, the mouse fibroblast cell line L929 was cultured on the artificial biomembranes at 37°C in a 5% CO ₂condition. For this, DMEM (Dulbecco's modified Eagles medium-high glucose, WelGENE, Korea) supplemented with 10% (v/v) FBS (fetal bovine serum, GIBCO), and 100 U streptomycin and 100 (µg/ml penicillin (GIBCO) was used. The artificial biomembrane was assayed for cell growth potential by MTT.

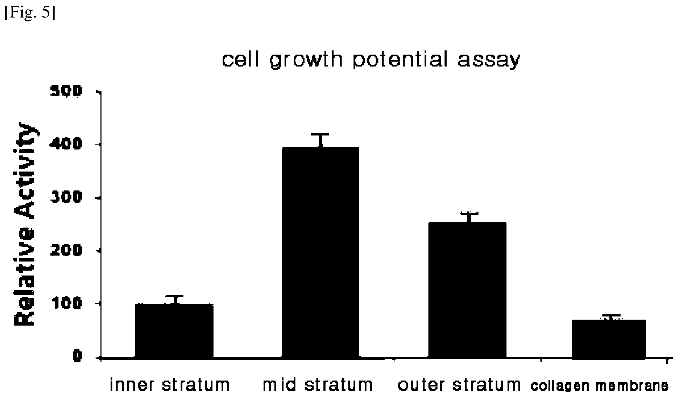

The results are depicted in FIG. 5 wherein cell growth potential (relative activity) is given according to cocoon stratum.

### 2. Test Result

As can be seen in FIG. 5, all the cocoon-based artificial biomembranes exhibited good cell growth potential, with 4-fold higher cell growth on the mid stratum than the inner stratum.

In other words, the mid stratum is more suitable than the inner stratum for use in a site that needs cell growth. In addition, the cocoon-based artificial biomembranes of the present invention were observed to grow the cells at higher efficiency, compared to the collagen membrane.

### <Description of Numerical References in Drawings>

10: Cocoon
11: cutting line 1
13: Inside surface
15: cutting line 2
17: cutting line 3
20: Cocoon fragment with a first thickness
30: Cocoon fragment with a second thickness
31: Inner stratum
33: Mid stratum
35: Outer stratum

## Claims

1. A silkworm cocoon-based artificial biomembrane comprising a fragment (20) of a silkworm cocoon (10), prepared by dividing a silkworm cocoon (10) into two or more fragments (20) in a predetermined form, the cocoon having a shell with a first thickness.

2. The silkworm cocoon-based artificial biomembrane of claim 1, wherein each of the fragments (20) is delaminated into a lamellar fragment (30) with a second thickness, the second thickness being smaller than the first thickness.

3. The silkworm cocoon-based artificial biomembrane of claim 2, wherein the lamellar fragment (30) with a second thickness is an inner stratum of the cocoon.

4. The silkworm cocoon-based artificial biomembrane of claim 2, wherein the lamellar fragment with a second thickness is a mid stratum of the cocoon.

5. The silkworm cocoon-based artificial biomembrane of claim 2, wherein the lamellar fragment with a second thickness is an outer stratum of the cocoon.

6. The silkworm cocoon-based artificial biomembrane of claim 3, wherein the lamellar fragment is sterilized.

7. The silkworm cocoon-based artificial biomembrane of claim 4, wherein the lamellar fragment is sterilized.

8. The silkworm cocoon-based artificial biomembrane of claim 5, wherein the lamellar fragment is sterilized.

9. The silkworm cocoon-based artificial biomembrane of any one of claims 6 to 8, wherein the lamellar fragment is packed.

10. A method for manufacturing a silkworm cocoon-based artificial biomembrane comprising a fragment of a silkworm cocoon, wherein the method comprises a first step of dividing a silkworm cocoon into two or more fragments in a predetermined form, the silkworm cocoon having a shell with a first thickness.

11. The method of claim 10, further comprising a second step of delaminating each of the fragments into a lamellar fragment with a second thickness, the second thickness being less than the first thickness.

12. The method of claim 11, further comprising a third step of packing the fragments of the second thickness prepared in the second step.

13. The method of claim 10 or 11, further comprising conducting sterilization before or after each step.

14. The method of claim 11 or 12, wherein the lamellar fragment with a second thickness is an inner stratum of the silkworm cocoon.

15. The method of claim 11 or 12, wherein the lamellar fragment with a second thickness is a mid stratum of the silkworm cocoon.

16. The method of claim 11 or 12, wherein the lamellar fragment with a second thickness is an outer stratum of the silkworm cocoon.

17. A silkworm cocoon-based artificial biomembrane according to any one of claims 1-9, for use in cell growth.

## Patentansprüche

1. Auf einem Seidenraupenkokon basierende, künstliche Biomembran, welche ein Fragment (20) eines Seidenraupenkokons (10) beinhaltet, welches vorbereitet wird, indem ein Seidenraupenkokon (10) in zwei Fragmente (20) oder mehr in einer vorbestimmten Form unterteilt wird, wobei der Seidenraupenkokon eine Schale mit einer ersten Dicke besitzt.

2. Auf einem Seidenraupenkokon basierende, künstliche Biomembran nach Anspruch 1, bei welcher jedes der Fragmente (20) in ein lamellares Fragment (30) mit einer zweiten Dicke delaminiert wird, wobei die zweite Dicke kleiner ist als die erste Dicke.

3. Auf einem Seidenraupenkokon basierende, künstliche Biomembran nach Anspruch 2, bei welcher das lamellare Fragment (30) mit einer zweiten Dicke eine innere Schicht des Kokons ist.

4. Auf einem Seidenraupenkokon basierende, künstliche Biomembran nach Anspruch 2, bei welcher das lamellare Fragment mit einer zweiten Dicke eine mittlere Schicht des Kokons ist.

5. Auf einem Seidenraupenkokon basierende, künstliche Biomembran nach Anspruch 2, bei welcher das lamellare Fragment mit einer zweiten Dicke eine äußere Schicht des Kokons ist.

6. Auf einem Seidenraupenkokon basierende, künstliche Biomembran nach Anspruch 3, bei welcher das lamellare Fragment sterilisiert ist.

7. Auf einem Seidenraupenkokon basierende, künstliche Biomembran nach Anspruch 4, bei welcher das lamellare Fragment sterilisiert ist.

8. Auf einem Seidenraupenkokon basierende, künstliche Biomembran nach Anspruch 5, bei welcher das lamellare Fragment sterilisiert ist.

9. Auf einem Seidenraupenkokon basierende, künstliche Biomembran nach einem der Ansprüche 6 bis 8, bei welcher das lamellare Fragment komprimiert ist.

10. Verfahren zum Herstellen einer auf einem Seidenraupenkokon basierenden, künstlichen Biomembran, welche ein Fragment eines Seidenraupenkokons beinhaltet, wobei das Verfahren einen Schritt des Unterteilens eines Seidenraupenkokons in zwei Fragmente oder mehr in einer vorbestimmten Form beinhaltet, wobei der Seidenraupenkokon eine Schale mit einer ersten Dicke besitzt.

11. Verfahren nach Anspruch 10, zudem beinhaltend einen zweiten Schritt des Delaminierens jedes der Fragmente in ein lamellares Fragment mit einer zweiten Dicke, wobei die zweite Dicke kleiner ist als die erste Dicke.

12. Verfahren nach Anspruch 11, zudem beinhaltend einen dritten Schritt des Komprimierens der Fragmente der zweiten Dicke, welche im zweiten Schritt vorbereitet wurden.

13. Verfahren nach Anspruch 10 oder 11, zudem beinhaltend Durchführen einer Sterilisation vor oder nach jedem Schritt.

14. Verfahren nach Anspruch 11 oder 12, bei welchem das lamellare Fragment mit einer zweiten Dicke eine innere Schicht des Seidenraupenkokons ist.

15. Verfahren nach Anspruch 11 oder 12, bei welchem das lamellare Fragment mit einer zweiten Dicke eine mittlere Schicht des Seidenraupenkokons ist.

16. Verfahren nach Anspruch 11 oder 12, bei welchem das lamellare Fragment mit einer zweiten Dicke eine äußere Schicht des Seidenraupenkokons ist.

17. Auf einem Seidenraupenkokon basierende, künstliche Biomembran nach einem der Ansprüche 1 bis 9 zum Gebrauch bei Zellwachstum.

## Revendications

1. Biomembrane artificielle à base de cocon de ver à soie, comprenant un fragment (20) d'un cocon de ver à soie (10), préparé en divisant un cocon de ver à soie (10) en deux fragments (20) ou plus dans une forme prédéterminée, le cocon présentant une coque avec une première épaisseur.

2. Biomembrane artificielle à base de cocon de ver à soie selon la revendication 1, dans laquelle chacun des fragments (20) est délaminé en un fragment lamellaire (30) avec une seconde épaisseur, la seconde épaisseur étant inférieure à la première épaisseur.

3. Biomembrane artificielle à base de cocon de ver à soie selon la revendication 2, dans laquelle le fragment lamellaire (30) avec une seconde épaisseur est une couche interne du cocon.

4. Biomembrane artificielle à base de cocon de ver à soie selon la revendication 2, dans laquelle le fragment lamellaire avec une seconde épaisseur est une couche moyenne du cocon.

5. Biomembrane artificielle à base de cocon de ver à soie selon la revendication 2, dans laquelle le fragment lamellaire avec une seconde épaisseur est une couche externe du cocon.

6. Biomembrane artificielle à base de cocon de ver à soie selon la revendication 3, dans laquelle le fragment lamellaire est stérilisé.

7. Biomembrane artificielle à base de cocon de ver à soie selon la revendication 4, dans laquelle le fragment lamellaire est stérilisé.

8. Biomembrane artificielle à base de cocon de ver à soie selon la revendication 5, dans laquelle le fragment lamellaire est stérilisé.

9. Biomembrane artificielle à base de cocon de ver à soie selon l'une quelconque des revendications 6 à 8, dans laquelle le fragment lamellaire est compacté.

10. Procédé de fabrication d'une biomembrane artificielle à base de cocon de ver à soie comprenant un fragment d'un cocon de ver à soie, dans lequel le procédé comprend une première étape consistant à diviser le cocon de ver à soie en deux fragments ou plus dans une forme prédéterminée, le cocon de ver à soie présentant une coque avec une première épaisseur.

11. Procédé selon la revendication 10, comprenant en outre une deuxième étape consistant à délaminer chacun des fragments en un fragment lamellaire avec une seconde épaisseur, la seconde épaisseur étant inférieure à la première épaisseur.

12. Procédé selon la revendication 11, comprenant en outre une troisième étape de compactage des fragments de la seconde épaisseur préparés dans la deuxième étape.

13. Procédé selon la revendication 10 ou 11, comprenant en outre la stérilisation avant ou après chaque étape.

14. Procédé selon les revendications 11 ou 12, dans lequel le fragment lamellaire avec une seconde épaisseur est une couche interne du cocon de ver à soie.

15. Procédé selon les revendications 11 ou 12, dans lequel le fragment lamellaire avec une seconde épaisseur est une couche centrale du cocon de ver à soie.

16. Procédé selon les revendications 11 ou 12, dans lequel le fragment lamellaire avec une seconde épaisseur est une couche externe du cocon de ver à soie.

17. Biomembrane artificielle à base de cocon de ver à soie selon l'une quelconque des revendications 1 à 9, pour utilisation dans la croissance cellulaire.
